# EUROPÄISCHE PATENTSCHRIFT

(11) **EP 1 397 499 B1**
(45) Veröffentlichungstag und Bekanntmachung des Hinweises auf die Patenterteilung: **23.12.2009**
(21) Anmeldenummer: 02737817.3
(22) Anmeldetag: 24.04.2002
(51) Int. Cl.: C12N 15/864

(54) **AAV-HELFERPLASMIDE ZUR HELFERVIRUS-FREIEN VERPACKUNG UND PSEUDOTYPISIERUNG VON AAV-VEKTOREN**
AAV HELPER PLASMIDS FOR HELPER VIRUS-FREE PACKAGING AND PSEUDO TYPIFICATION OF AAV VECTORS
PLASMIDES AUXILIAIRES AAV PERMETTANT L'ENCAPSIDATION SANS VIRUS AUXILIAIRE ET LE PSEUDOTYPAGE DE VECTEURS AAV

(30) Priorität: 25.04.2001 DE 10120265
(43) Veröffentlichungstag der Anmeldung: 17.03.2004
(73) Patentinhaber: Deutsches Krebsforschungszentrum Stiftung des öffentlichen Rechts, 69120 Heidelberg (DE)
(72) Erfinder: GRIMM, Dirk, Palo Alto, CA 94303 (US); KLEINSCHMIDT, Jürgen, 69245 Bammental (DE)
(74) Vertreter: Schüssler, Andrea
(86) Internationale Anmeldenummer: PCT/DE2002/001502
(87) Internationale Veröffentlichungsnummer: WO 2002/088347

(56) Entgegenhaltungen:
- WO-A-99/47691
- DE-A- 19 644 500
- GRIMM D ET AL: "Novel tools for production and purification of recombinant adenoassociated virus vectors" HUMAN GENE THERAPY, XX, XX, Bd. 9, Nr. 18, 10. Dezember 1998 (1998-12-10), Seiten 2745-2760, XP002093963 ISSN: 1043-0342
- COLLACO R F ET AL: "A helper virus-free packaging system for recombinant adeno-associated virus vectors" GENE: AN INTERNATIONAL JOURNAL ON GENES AND GENOMES, ELSEVIER SCIENCE PUBLISHERS, BARKING, GB, Bd. 238, Nr. 2, 1. Oktober 1999 (1999-10-01), Seiten 397-405, XP002200459 ISSN: 0378-1119
- TAL J: "ADENO-ASSOCIATED VIRUS-BASED VECTORS IN GENE THERAPY" JOURNAL OF BIOMEDICAL SCIENCE, KARGER, BASEL, CH, Bd. 7, Nr. 4, Juli 2000 (2000-07), Seiten 279-291, XP000994813 ISSN: 1021-7770
- GRIMM DIRK ET AL: "Progress in adeno-associated virus type 2 vector production: Promises and prospects for clinical use" HUMAN GENE THERAPY, XX, XX, Bd. 10, Nr. 15, 10. Oktober 1999 (1999-10-10), Seiten 2445-2450, XP002200460 ISSN: 1043-0342
- GRIMM DIRK ET AL: "Helper virus-free, optically controllable, and two-plasmid-based production of adeno-associated virus vectors of serotypes 1 to 6." MOLECULAR THERAPY: THE JOURNAL OF THE AMERICAN SOCIETY OF GENE THERAPY. UNITED STATES JUN 2003, Bd. 7, Nr. 6, Juni 2003 (2003-06), Seiten 839-850, XP002256403 ISSN: 1525-0016
- ZOLOTUKHIN SERGEI ET AL: "Production and purification of serotype 1, 2, and 5 recombinant adeno-associated viral vectors." METHODS (ORLANDO), Bd. 28, Nr. 2, 20. Oktober 2002 (2002-10-20), Seiten 158-167, XP002256404 ISSN: 1046-2023

## Beschreibung

Die vorliegende Erfindung betrifft AAV-Helferplasmide zur Helfervirus-freien Verpackung und Pseudotypisierung von AAV-Vektoren. Diese AAV-Helferplasmide beinhalten folgende DNA-Sequenzen: (a1) Das *rep*-Gen von AAV-2 und (a2) das *cap*-Gen von AAV-1, AAV-3, AAV-4, AAV-5 oder AAV-6, oder (b) das *cap*-Gen und das *rep*-Gen von jeweils AAV-1, AAV-3, AAV-4, AAV-5 oder AAV-6, und (c) alle weiteren Helfervirus-DNA-Sequenzen, die für eine Ausbildung von AAV-Partikeln notwendig sind, wobei diese Helfervirus-DNA-Sequenzen die Ad5-Gene E2A, E4 und VA sind. Die Erfindung betrifft ferner die Verwendung dieser AAV-Helferplasmide bzw. AAV-Partikel mit einer von diesen AAV-Helferplasmiden kodierten Hülle und einem AAV-Expressionsvektor zur Gentherapie.

AAVs sind einzelsträngige, zur Familie der Parvorviren gehörende DNA-Viren. Für ihre Replikation benötigen AAVs Helferviren, insbesondere Adenoviren oder Herpesviren. In Abwesenheit von Helferviren integrieren AAVs in das Wirtszellgenom, insbesondere an einer spezifischen Stelle von Chromosom 19. Das Genom von AAVs ist linear und weist eine Länge von ca. 4680 Nukleotiden auf. Dieses umfaßt zwei Leserahmen, die für ein strukturelles und ein nichtstrukturelles Gen codieren. Das strukturelle Gen wird als *cap*-Gen bezeichnet. Dieses steht unter der Kontrolle des P40-Promotors und kodiert für drei Capsid-Proteine. Das nichtstrukturelle Gen wird mit *rep*-Gen bezeichnet und kodiert für die Rep-Proteine Rep 78, Rep 68, Rep 52 und Rep 40. Die beiden ersteren werden unter der Kontrolle des P5 Promotors exprimiert, während die Expression von Rep 52 und Rep 40 unter der Kontrolle des P19-Promotors steht. Die Funktionen der Rep-Proteine liegen u.a. in der Regulation der Replikation und Transkription des AAV-Genoms.

AAVs werden schon seit einiger Zeit intensiv als mögliche Vektoren für die humane Gentherapie entwickelt und getestet. Unter den sechs verschiedenen Serotypen von AAV (AAV-1 bis AAV-6), die bisher kloniert und sequenziert wurden, stellt AAV-2 den am besten charakterisierten Serotyp dar und die meisten zur Zeit verwendeten Vektoren basieren auf AAV-2. In den letzten Jahren wurden jedoch auch Berichte über die Erzeugung und Evaluierung der anderen fünf AAV-Serotypen veröffentlicht. Es stellte sich heraus, daß die ITRs (inverted terminal repeats) an jedem Ende des AAV-Genoms die einzigen *cis*-Elemente sind, die für die Vermehrung (d.h. Exzision der viralen DNA vom Plasmid, Replikation und Verpackung der intermediären DNA-Sequenzen) von AAV-Vektoren (mit Hilfe des Helfervirus) erforderlich sind. Somit war es naheliegend, daß im Prinzip jede DNA, die von AAV-ITRs flankiert ist und hinsichtlich der Länge mit einem Wildtyp-Virusgenom vergleichbar ist, in AAV-Kapside in Gegenwart der *rep*- und *cap*-Genprodukte *in trans* sowie der Helfervirusfunktionen verpackt werden kann. In den meisten Fällen wurde für die Herstellung dieser Vektoren ein Verfahren angewandt, bei dem Helferviren verwendet werden müssen, d.h., die Zellen werden mit dem AAV-Vektor und Helferplasmiden cotransfiziert und danach mit dem Helfer-Adenovirus infiziert was zu rekombinanten AAV-Vektoren führt, die allerdings mit Adenovirus kontaminiert sind. Eine weitere Strategie beruht auf einer Dreifach-Transfektion, bei der zusätzlich zur Vermeidung einer Kontamination mit Helferviren zur Bereitstellung von Helferfunktionen ein nicht-infektiöses adenovirales Plasmid verwendet wird.

Zusammengefaßt gibt es zur Zeit drei unterschiedliche Ansätze: (a) Cotransfektion von AAV-Helfersequenzen, die das *rep*- und *cap-*Gen des jeweiligen AAV-Serotyps zur Verfügung stellen, sowie den entsprechenden Vektorplasmiden von AAV-2, AAV-3, AAV-5 oder AAV-6, (b) Cotransfektion von AAV-2 Vektorplasmiden mit AAV-Helferplasmiden, die das *rep*-Gen von AAV-2 und das *cap*-Gen von AAV-1, AAV-3 oder AAV-5 tragen, und (c) Cotransfektion von AAV-2-Vektorplasmiden mit *rep*-*cap*-Genen von AAV-1, AAV-3, AAV-4 oder AAV-6. Die adenoviralen Helferfunktionen werden bei allen drei Ansätzen durch Infektion mit Adenoviren oder durch zusätzliche Transfektion von Plasmiden, die das adenovirale Genom tragen, zur Verfügung gestellt. Allerdings weisen alle bisherigen Ansätze bestimmte gravierende Nachteile auf. So müssen (a) entweder verschiedene Vektorplasmide für die Verpackung in verschiedene AAV-Serotypen verwendet werden, (b) die Vektorproduktion durch Tripelinfektionen ist umständlich und teuer und (c) bei der Doppeltransfektion und Infektion mit Adenoviren ergibt sich das Problem der Kontamination mit Adenoviren.

Das der vorliegenden Erfindung zugrunde liegende technische Problem war somit, ein Verfahren zur Verpackung von AAV-Vektoren bereitzustellen, das die vorstehend diskutierten Nachteile nicht aufweist, d.h. eine Helfervirus-freie Verpackung von AAV-Vektor-DNA in ein gewünschtes AAV-Kapsid durch einfache Cotransfektion mit einem geeigneten Helfer/Verpackungsplasmid erlaubt, wobei keine Kontamination mit Adenoviren eintritt.

Die Lösung dieses technischen Problems erfolgt durch die Bereitstellung der in den Ansprüchen gekennzeichneten Ausführungsformen. Es wurde überraschenderweise festgestellt, daß das technische Problem durch die Verwendung eines Helferplasmids gelöst werden konnte, das die vollständigen Helferfunktionen für die Verpackung des Vektorplasmids abgeleitet von AAV, vorzugsweise AAV-2, in das gewünschte AAV-Kapsid erlaubt. Der Hauptvorteil ist dabei die Vereinfachung der Herstellung von pseudotypisierten AAV-Vektoren, die darüber hinaus frei von einer Kontamination mit Adenoviren sind. Bei diesem einfachen Verfahren wurde von dem in der deutschen Patentanmeldung 196 44 500.0-41 beschriebenen Helferplasmid pDG ausgegangen, das alle AAV-2 und adenoviralen Gene besitzt, deren Produkte für die Erzeugung von AAV-2-Vektoren erforderlich sind. Dazu wurde das *cap*-Gen des AAV-Serotyps 2 auf diesem Plasmid jeweils gegen ein *cap*-Gen des Serotyps 1, 3, 4, 5 oder 6 ausgetauscht, wobei insgesamt fünf neue Helferplasmide mit den Bezeichnungen pDP1, pDP3, pDP4, pDP5 bzw. pDP6 erhalten wurden. Im Falle von pDP4 hat es sich als besonders günstig erwiesen, auch das rep-Gen von AAV-2 gegen das rep-Gen von AAV-4 auszutauschen. Die Cotransfektion eines AAV-2-Vektorplasmids mit dem jeweiligen Helferplasmid ergab rekombinante AAV-Partikel, die aus dem AAV-2-Vektor bestanden, der in AAV-Kapsidhüllen entsprechend dem Serotyp des verwendeten pDP-abgeleiteten Helferplasmids entsprachen. Die verschiedenen Vektor-Stammlösungen wurden hinsichtlich der Titer an total assemblierten, DNA enthaltenden sowie infektiösen Partikeln analysiert und die unterschiedlichen Effizienzen der Vektorproduktion miteinander verglichen. Dabei ist festzuhalten, daß alle rekombinanten AAV-Stammlösungen frei von Kontaminationen mit Wildtyp-AAV waren. Darüber hinaus kann in jedes Helferplasmid eine Expressionskassette inseriert werden, die z.B. das Gen für das "red fluorescent" Protein ("Dsred", Clontech, Palo Alto, USA) unter Kontrolle des RSV-Promotors enthält und somit können nach Anregung mit einer geeigneten Wellenlänge die erfolgreich transfizierten Zellen anhand der leuchtenden roten Farbe,leicht und schnell nachgewiesen werden.

Gegenstand der vorliegenden Erfindung ist somit ein AAV-Helferplasmid, das folgende DNA-Sequenzen umfaßt:
(a1) Das *rep*-Gen von AAV-2; und
(a2) das *cap*-Gen von AAV-1, AAV-3, AAV-4, AAV-5 oder AAV-6; oder
(b) das *cap*-Gen und das *rep*-Gen von jeweils AAV-1, AAV-3, AAV-4, AAV-5 oder AAV-6; und
(c) alle weiteren Helfervirus-DNA-Sequenzen, die für eine Ausbildung von AAV-Partikeln notwendig sind, wobei diese Helfervirus-DNA-Sequenzen die Ad5-Gene E2A, E4 und VA sind.

Der hier verwendete Ausdruck "Helfervirus-DNA-Sequenzen" betrifft alle DNA-Sequenzen eines Helfervirus, die zur Ausbildung von AAV-Partikeln notwendig sind.

Das erfindungsgemäße AAV-Helferplasmid enthält als Helfervirus-DNA-Sequenzen die Ad5-Gene E2A, E4 und VA, die z.B. von dem in der deutschen Patentanmeldung 196 44 500.0-41 beschriebenen Plasmid pDG stammen können und wobei diese unter der Kontrolle des jeweiligen ursprünglichen Promotors stehen oder unter der Kontrolle heterologer Promotoren.

Der hier verwendete Ausdruck "AAV-Helferplasmid" betrifft nicht nur Helferplasmide mit den ursprünglichen unter (a) bis (c) aufgelisteten Genen, sondern auch Helferplasmide mit veränderten Genen, die z.B. Deletionen oder Insertionen von Nukleotiden aufweisen, jedoch noch Proteine mit der gewünschten biologischen Funktion kodieren. Der Fachmann kann mittels gängiger Verfahren bestimmen, ob ein verändertes Gen noch ein Produkt mit der gewünschten biologischen Funktion kodiert. Der Fachmann kennt auch Quellen für die einzelnen Gene, die das erfindungsgemäße AAV-Helferplasmid auszeichnen. Allgemeine, auf dem Fachgebiet bekannte Verfahren können zur Konstruktion von AAV-Helferplasmiden, die die vorstehenden DNA-Sequenzen und gegebenenfalls weitere Sequenzen enthalten, verwendet werden. Zu diesen Verfahren zählen beispielsweise in vitro-Rekombinationstechniken, synthetische Verfahren, sowie in vivo-Rekombinationsverfahren, wie sie beispielsweise in Sambrook et al., Molecular Cloning: A Laboratory Manual, 2. Ausgabe, Cold Spring Harbor Laboratory Press, Cold Spring Harbor NY (1989))., beschrieben sind. Beispielsweise kann als Grundgerüst für ein erfindungsgemäßes Helferplasmid das in der deutschen Patentanmeldung 196 44 500.0-41 beschriebene Plasmid pDG verwendet werden, bei dem z.B. über PCR und geeignete Primer das ursprüngliche AAV-2 *cap*-Gen gegen ein *cap*-Gen von AAV-1, AAV-3, AAV-4, AAV-5 oder AAV-6 (AAV-1, Xiao et al., J. Virol. 73 (1999), 3994-4003; AAV-3, Muramatsu et al., Virol. 221 (1996), 208-217; AAV-4, Chiorini et al., J. Virol. (1997), 6823-6833; AAV-5, Bantel-Schaal et al., J. Virol. 73 (1999), 939-947; Chiorini et al., J. Virol. 73 (1999), 1303-1319; AAV-6, Rutledge et al., J. Virol. 72 (1998), 309-319) ausgetauscht wird.

In einer mehr bevorzugten Ausführungsform enthält das erfindungsgemäße AAV-Helferplasmid zusätzlich eine Expressionskassette zur Expression eines Markerproteins, vorzugsweise eines Fluoreszenzproteins. In diesem Zusammenhang bedeutet der Begriff "Expressionskassette" eine Kombination aus einem z.B. ein Fluoreszenzgen kodierenden Gen, das unter der Kontrolle eines geeigneten Promotors steht. Dies erlaubt den einfachen Nachweis einer Transfektion der gewünschten Zielzelle. Beispiele für geeignete Fluoreszenzproteine kodierende Gene sind das rfp-(red), gfp-(green), cfp-(cyan), yfp-(yellow) und Luciferase-kodierende Gen und Beispiele für geeignete Promotoren sind RSV (Rous Sarcoma Virus)-Promotor, CMV (Cytomegalo Virus)-Promotor und HSV (Herpes Simplex Virus)-tk Promotor. Diese Expressionskassette wird in das AAV Helferplasmid, vorzugsweise in die ClaI-Schnittzelle zwischen dem Ende des cap-Gens und dem Beginn des adenoviralen VA-Gens eingefügt. Diese ClaI-Schnittstelle ist in pDG und in pDP1, pDP3, pDP4, pDP5 sowie pDP6 vorhanden.

Folgende AAV-Helferplasmide wurden bei der DSMZ, Braunschweig, gemäß den Bestimmungen des Budapester Vertrags am 23. April 2001 hinterlegt: pDP1 mit der Hinterlegungsnummer DSM 14256, pDP3 mit der Hinterlegungsnummer DSM 14255, pDP4 mit der Hinterlegungsnummer DSM 14254, pDP5 mit der Hinterlegungsnummer DSM 14253 und pDP6 mit der Hinterlegungsnummer DSM 14252.

Gegenstand der vorliegenden Erfindung sind ferner AAV-Partikel, deren Kapsidhülle von einem erfindungsgemäßen AAV-Helferplasmid kodiert ist und das einen AAV-Expressionsvektor enthält. Geeignete AAV-Expressionsvektoren sind dem Fachmann bekannt (Zolotukhin et al., J. Virol. 70 (1996), 4646-4653). Vorzugsweise handelt es sich bei diesem AAV-Expressionsvektor um einen Expressionsvektor, der mindestens folgende DNA-Sequenzen umfaßt: (a) die 5'ITR und 3'ITR eines AAV-2; (b) einen in Säugern aktiven konstitutiven oder induzierbaren Promotor und (c) ein Poly-Adenylierungssignal. Dabei umfaßt der Begriff "5'ITR" bzw. "3'ITR" alle "5'ITR"- bzw. "3'ITR"-Sequenzen, die dem Vektor die Integration in das Wirtsgenom erlauben. Der hier verwendete Ausdruck "ein in Säugern aktiver konstitutiver oder induzierbarer Promotor" umfaßt alle Promotoren, die in Säugern die Transkription der gewünschten DNA-Sequenz erlauben, vor allem solche, die zu einer starken Expression führen, vorzugsweise heterologe Promotoren. Geeignete Promotoren sind dem Fachmann bekannt und umfassen beispielsweise die konstitutiven Promotoren CMV- und Cytokeratin K14-Promotoren oder die induzierbaren Promotoren MMTV-(Mouse Mammary Tumor Virus), Metallothionein- und Tetrazyklin-regulierbare Promotersysteme (Tet-on/-off). Der AAV-Expressionsvektor kann darüber hinaus das gewünschte, in den Säugerzellen zu exprimierende Gen, dessen Expression z.B. für eine Gentherapie wünschenswert ist, enthalten. Ferner kann der AAV-Expressionsvektor ein Gen enthalten, das einen nachweisbaren phänotypischen Marker codiert, und somit das erfolgreiche Einschleußen des AAV-Expressionsvektors in die Zielzelle nachgewiesen werden kann. Geeignete Markergene sind z.B. die vorstehend genannten.

Erfindungsgemäße AAV-Partikel können durch geeignete Verfahren erhalten werden, z.B. durch Cotransfektion von Säugerzellen, z.B. COS-Zellen oder 293-Zellen, mit einem erfindungsgemäßen AAV-Helferplasmid und einem vorstehend beschriebenen AAV-Expressionsvektor, z.B. mittels des in dem nachstehenden Beispiel 2 beschriebenen Verfahrens. Der erzielbare Titer beträgt in der Regel zwischen 10⁶ und 10⁸ Virus-Partikel/ml.

Mit einem erfindungsgemäßen AAV-Helferplasmid bzw. AAV-Partikel kann eine Gentherapie durchgeführt werden, wobei die Zellen durch übliche Verfahren transfiziert werden. Als Transfektionstechniken sind z.B. Elektroporation, Lipofektion und vorzugsweise Calciumphosphat-Präzipitation zu nennen. Die Zellen können in einem Organismus vorliegen, andererseits können die zu transfizierenden Zellen auch aus einem Organismus isoliert, außerhalb des Organismus transfiziert und dann wieder in den Organismus zurückgeführt werden. Solche Zellen werden als autologe Zellen bezeichnet, Desweiteren können hinsichtlich des Organismus auch allogene Zellen zur Transduktion verwendet werden. Hierbei ist es günstig, wenn diese Zellen einem dem Organismus entsprechenden HLA-Typ angehören. Der Fachmann kennt Verfahren, Zellen einen bestimmten HLA-Typ zu verleihen.

Die vorliegende Erfindung betrifft auch die vorstehend beschriebenen AAV-Helferplasmide und AAV-Expressionsvektoren enthaltende Wirtszellen, die beispielsweise zur Erzeugung und Gewinnung von AAV-Partikeln dienen können. Zu diesen Wirtszellen zählen Säugerzellen, vorzugsweise 293-, 911- oder PerC6-Zellen. Verfahren zur Transfektion dieser Wirtszellen, zur phänotypischen Selektion von Transfektanten etc. sind dem Fachmann bekannt. Der Fachmann kennt auch geeignete Züchtungsverfahren und Medien, um Säugerzellen kultivieren zu können. Das Medium für die Züchtung kann jedes Medium sein, das üblicherweise für die Züchtung von Säugerzellen verwendet wird, z.B. IMEM, DMEM etc. Die Zellen werden in dem vorstehenden Medium unter geeigneten Bedingungen, gegebenenfalls unter (teilweiser) Erneuerung des Mediums in geeigneten Zeitabständen, gezüchtet. Geeignete Bedingungen, beispielsweise hinsichtlich geeigneter Behälter, Temperatur, relativer Luftfeuchte, O₂-Gehalt und CO₂-Gehalt der Gasphase sind dem Fachmann bekannt. Vorzugsweise werden die Zellen in dem vorstehenden Medium unter den folgenden Bedingungen kultiviert: (a) 37°C, (b) 100% rel. Luftfeuchte, (c) 10% O₂ und (d) 5% bis 7% CO₂. Die Gewinnung der AAV-Partikel, vorzugsweise aus dem Kulturüberstand, kann durch übliche Standardverfahren erfolgen, z.B. Frier-Tau-Lyse, Filtration, Zentrifugation und chromatographische Trennung und Konzentrierung. Vorzugsweise werden die AAV-Partikel, vor allem für eine Anwendung im klinischen Bereich, noch weiter gereinigt, beispielsweise über Ionenaustausch-Chromatographie und Heparin-Affinitäts-Chromatographie.

Gegenstand der vorliegenden Erfindung ist auch ein Arzneimittel, das ein erfindungsgemäßes AAV-Helferplasmid bzw. AAV-Partikel enthält. Hierbei kann das Arzneimittel zusätzlich einen pharmazeutisch verträglichen Träger enthalten. Geeignete Träger und die Formulierung derartiger Arzneimittel sind dem Fachmann bekannt. Zu geeigneten Trägern zählen beispielsweise Phosphatgepufferte Kochsalzlösungen, Wasser, Emulsionen, beispielsweise Öl/Wasser-Emulsionen, Netzmittel, sterile Lösungen etc. Die Art des Trägers hängt davon ab, wie das erfindungsgemäße AAV-Helferplasmid bzw. AAV-Partikel verabreicht werden soll. Die geeignete Dosierung wird von dem behandelnden Arzt bestimmt und hängt von verschiedenen Faktoren ab, beispielsweise von dem Alter, dem Geschlecht, dem Gewicht des Patienten, dem Schweregrad der Erkrankung, der Art der Verabreichung etc.. Es hat sich dabei gezeigt, daß mit erfindungsgemäßen AAV-Partikeln hohe Transduktionsraten bei unterschiedlichsten Zellen, z.B. primären Zellen des Augen-Hornhautepithels oder Muskelzellen, erreicht werden.

### Kurze Beschreibung der Figuren

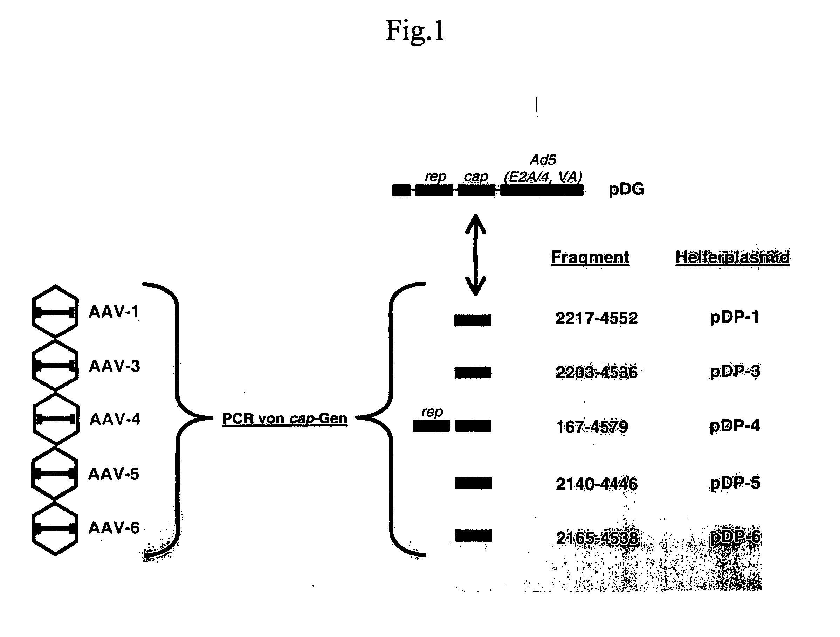

Figur 1: PCR-Stragie zur Erzeugung der AAV-Helferplasmide pDP1 und pDP3 bis pDP6
   Die in der Spalte "Fragment" angegebenen Zahlenwerte bezeichnen die ersten bzw. letzten in den jeweiligen *cap*- (bzw. *rep*- und *cap*-Genen) enthaltenen Basenpaare. Die Zahlen beziehen sich dabei auf das Genom des jeweiligen Serotyps.

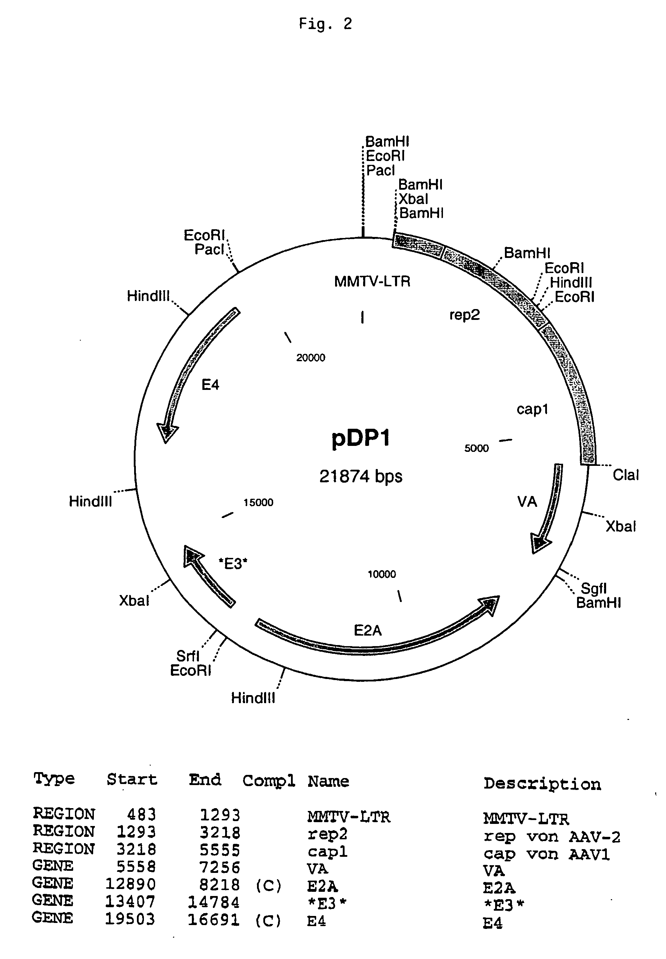

Figur 2: Physikalische Karte des AAV-Helferplasmids pDP1
   Positionen 483-1293: MMTV-LTR, 1293-3218: *rep*-Gen von AAV-2, 3219-5555: *cap*-Gen von AAV-1, 5558-7256: VA, 12890-8218 (C) (C = komplementär gegenläufig): E2A, 13407-14784: *E3* (*E3* = Rest der E3-Region), 19503-16691 (C): E4.

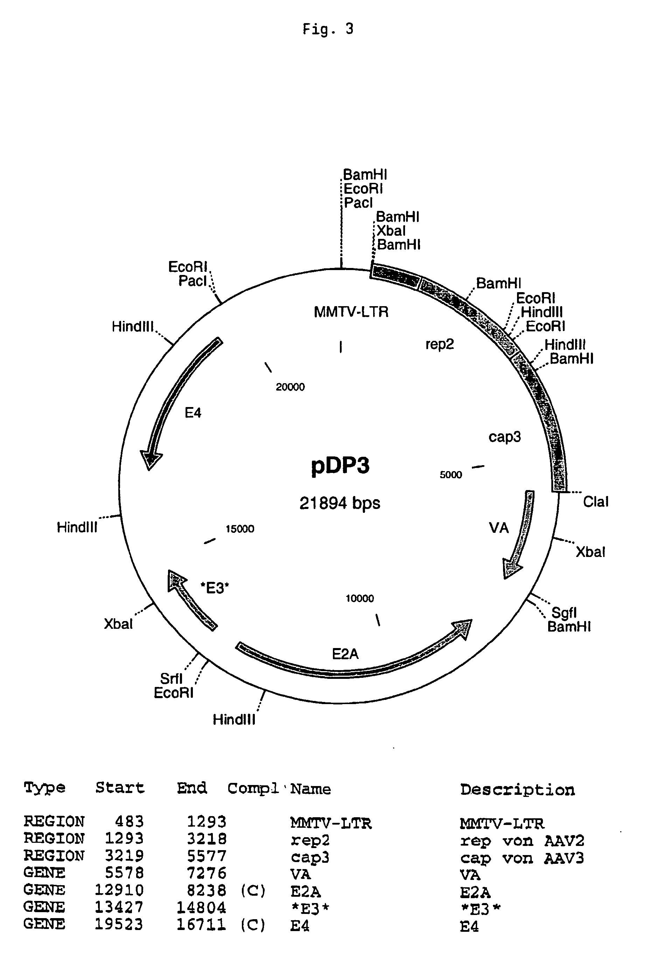

Figur 3: Physikalische Karte des AAV-Helferplasmids pDP3
   Positionen 483-1293: MMTV-LTR, 1294-3218: *rep*-Gen von AAVs-2, 3219-5577: *cap*-Gen von AAV-3, 5558-7276: VA, 12910-8238 (C): E2A, 13427-14804: *E3* 19523-16711 (C): E4.

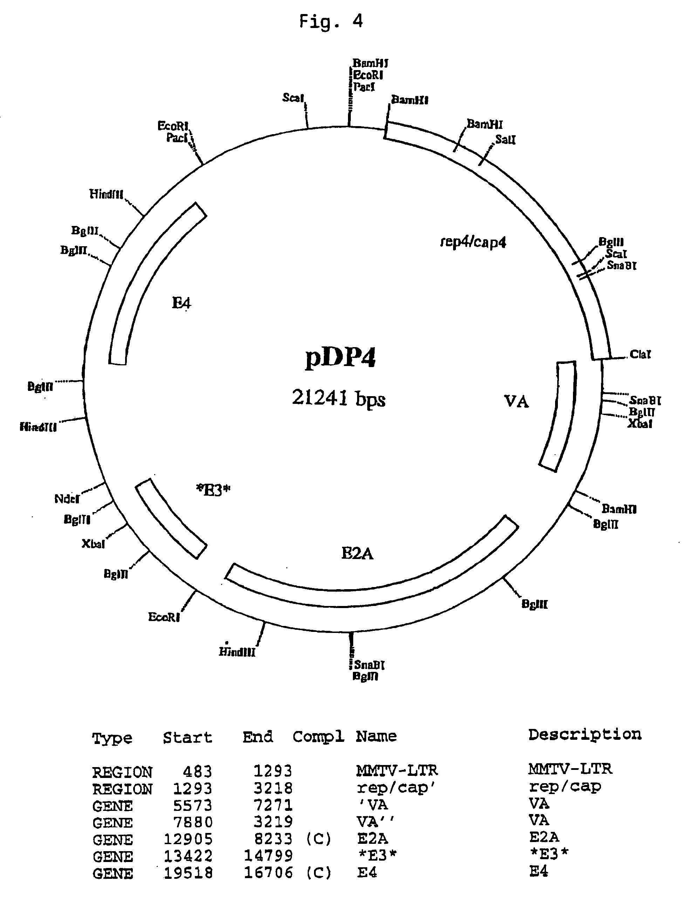

Figur 4: Physikalische Karte des AAV-Helferplasmids pDP4
   Positionen 483-1293: MMTV-LTR, 1293-3218: *rep*/*cap*-Gen 5573-7271: VA, 7880-3219: VA, 12905-8233 (C): E2A, 13422-14799: *E3* 19518-16706 (C): E4.

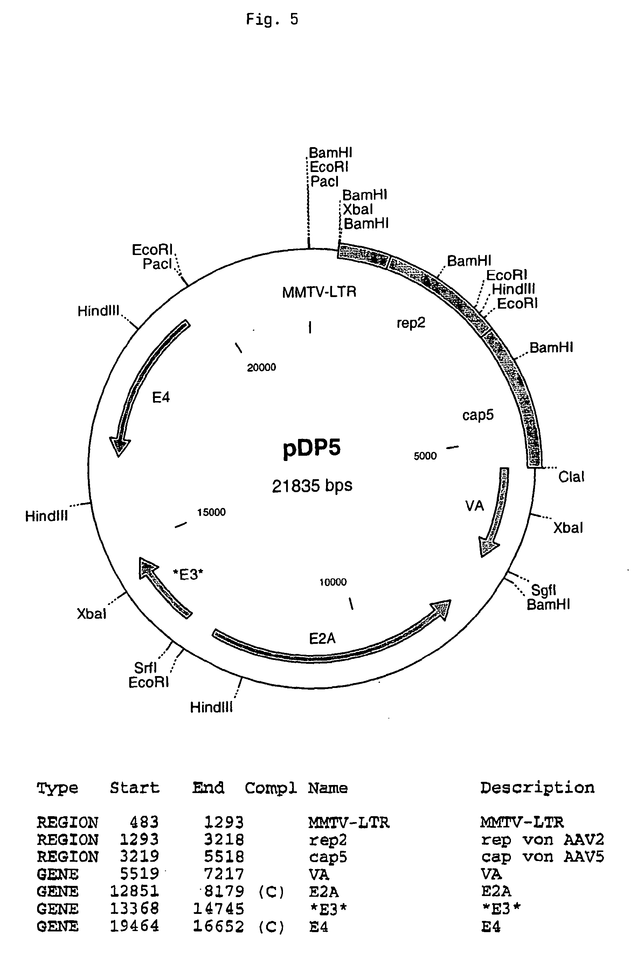

Figur 5: Physikalische Karte des AAV-Helferplasmids pDP5
   Positionen 483-1293: MMTV-LTR, 1294-3218: *rep*-Gen von AAV-2, 3219-5518: *cap*-Gen von AAV-5, 5519-7217: VA, 12851-8179 (C): E2A, 13368-14745: *E3* 19464-16652 (C): E4.

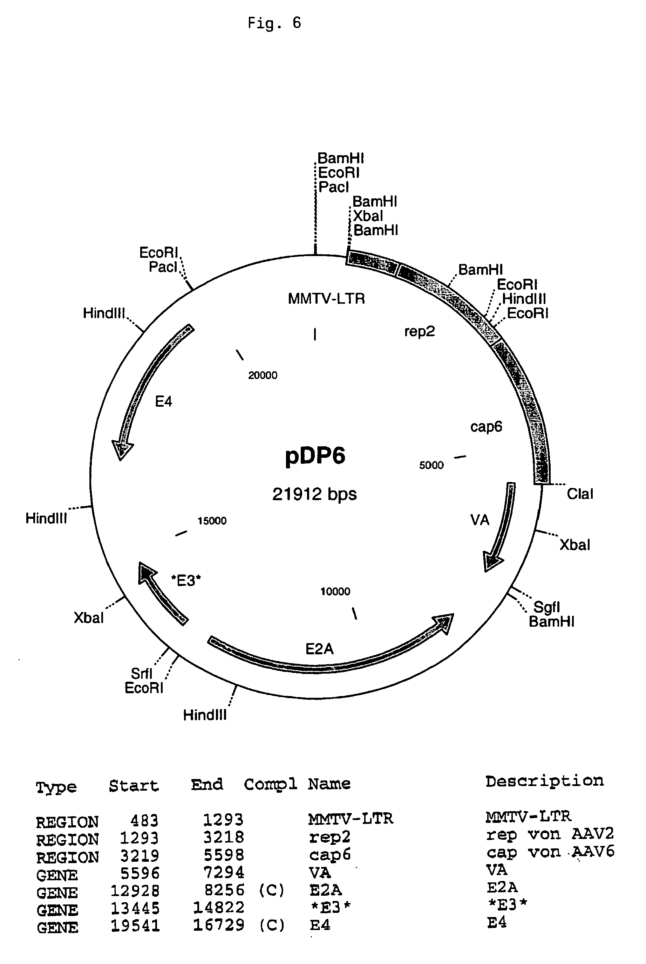

Figur 6: Physikalische Karte des AAV-Helferplasmids pDP6
   Positionen 483-1293: MMTV-LTR, 1294-3218: *rep*-Gen von AAV-2, 3219-5598: *cap*-Gen von AAV-3, 5596-7294: VA, 12928-8256 (C): E2A, 13445-14822: *E3*, 19541-16729 (C): E4.
Figur 7: Produktion pseudotypisierter AAV-2-Vektoren
   Im oberen Teil der Abbildung sind schematisch die zur Titration der in verschiedene Kapside verpackten AAV-2 Vektoren dargestellt (Grimm et al. Gene Therapy, 6 (1999), 1322-1330).

Im unteren Teil der Abbildung sind repräsentative Titer von AAV-2 Vektoren, die mit dem beschriebenen Verfahren in Kapside von AAV-2 bzw. in Kapside von AAV-1, -3, -4, -5, oder AAV-6 verpackt wurden, dargestellt.

Die Erfindung wird durch die nachfolgenden Beispiele erläutert

### Beispiel 1

### Herstellung der AAV-Helferplasmide pDP1 und pDP3 bis pDP6

Zur Herstellung der AAV-Helferplasmide pDP1 und pDP3 bis pDP6 wurde von dem in der deutschen Patentanmeldung 196 44 500.0-41 beschriebenen Plasmid pDG ausgegangen. Dieses besitzt eine Gesamtlänge von 21846 Basenpaaren. Das in pDG enthaltene AAV-Genom besitzt einschließlich des den AAV-2 p5 Promotor ersetzenden MMTV-Promotors insgesamt eine Länge von 5044 Basenpaaren.

Zunächst wurden 293-Zellen mit den verschiedenen Serotypen und Ad-5 als Helfervirus unter Standardbedingungen infiziert. Nach drei Tagen wurde replizierte virale AAV-DNA aus den Zellen isoliert und aufgereinigt. Diese DNA diente dann als Matrize in einer PCR-Reaktion zur Amplifikation der *cap*-Gene der jeweiligen Serotypen (Tabelle 1). Im Fall von AAV-4 zur Herstellung von pDP4 wurde außerdem das *rep*-Gen amplifiziert. Die Primer für die verschiedenen PCR-Reaktionen wurden dabei so gewählt, daß am 3'-Ende der jeweiligen Produkte eine Schnittstelle für das Restriktionsenzym ClaI erhalten wurde, das linke Ende war dagegen glatt (d.h. ohne Schnittstelle bzw. überstehendes Ende). Zur Klonierung der PCR-Fragmente wurde das Plasmid pDG mit den Enzymen SwaI (das ohne überstehende Enden schneidet) und ClaI linearisiert wodurch das ursprünglich in Plasmid pDG enthaltene *cap*-Gen von AAV-2 entfernt wurde. An dessen Stelle wurden dann die jeweiligen *cap*-Gene der anderen AAV-Serotypen einkloniert. Im Fall von AAV-4 wurden das *rep-* und *cap*-Gen amplifiziert. Am linken Ende des *rep-*Gens wurde dabei eine BlnI-Erkennungsstelle aus dem AAV-4-Genom mit amplifiziert. Nach entsprechender Restriktion erlaubte dies die Klonierung des AAV-4 *rep-*Gens zusammen mit dem AAV-4 *cap*-Gen (wobei am 3'-Ende wiederum mit wie vorstehend beschrieben mit ClaI geschnitten wurde) in das mit XbaI (kompatibel zu BlnI) und ClaI geschnittene Plasmid pDG. Durch den Vardau mit XbaI und ClaI werden aus dem Plasmid pDG sowohl das *rep-* als auch das *cap*-Gen von AAV-2 entfernt.

### Beispiel 2

### Herstellung pseudotypisierter AAV-2-Vektoren

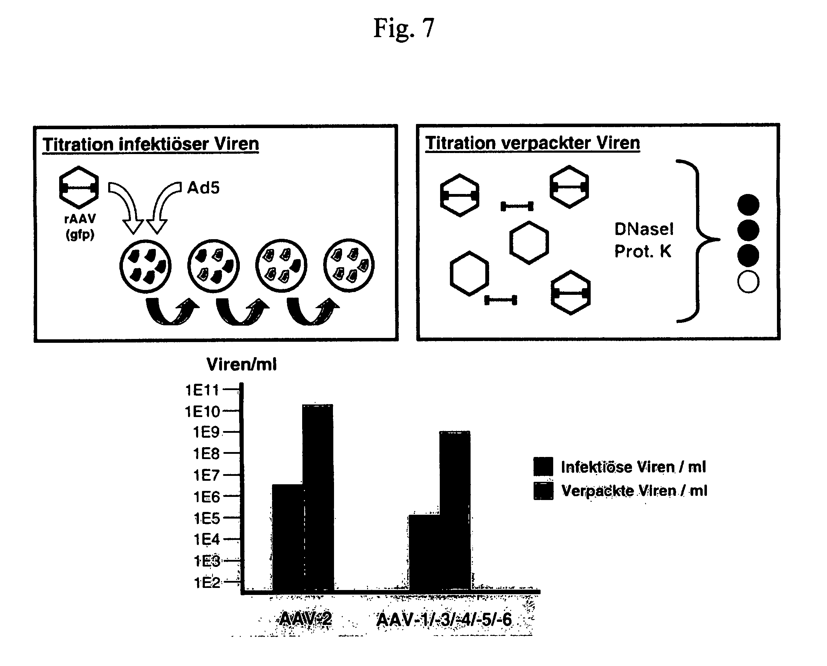

Zur (schematisch in Figur 7 dargestellten) Produktion pseudotypisierter AAV-2-Vekoren wurden 293T-Zellen mit dem AAV-2-Vektorplasmid pTRUF5 (Zolotukhin et al., J. Virol. 70 (1996), 4646-4654) und je einem der 6 verschiedenen AAV-Helferplasmide (pDG, pDP1, pDP3, pDP4, pDP5 und pDP6) cotransfiziert, d.h. in allen Fällen war das verwendete Vektorplasmid identisch und lediglich das AAV-Helferplasmid variierte. Nach drei Tagen Inkubation wurden Rohüberstände aus den Zellen durch Frier/Auftau-Lysaten gewonnen und die erhaltenen Viren quantifiziert. Typischerweise enthält ein Überstand aus Zellen, die mit einem Vektor und einem erfindungsgemäßen AAV-Helferplasmid cotransfiziert worden waren, zwischen 10⁶ und 10⁷ infektiöse Partikel pro ml. Die Titration der infektiösen Viren bzw. verpackter Genome erfolgte nach dem in Grimm et al., Gene Therapy 6 (1999), 1322-1330, beschriebenen Verfahren.

**TABELLE 1**

| Zur Amplifikation der AAV cap- (und AAV-4 rep-) Gene verwendete Oligonukleotide | | |
|---|---|---|
| AAV-1: | (links) | 5'- CCAGGTATGGCTGCCGATGGTTATC -3' |
| | (rechts) | 5'- GTCCAATCGATGCGAAGCGCAACCAAGCAG -3' |
| | | |
| AAV-3: | (links) | 5'-CCAGGTATGGCTGCTGACGGTTATC -3' |
| | (rechts) | 5'- GTCCAATCGATGCAGTTGTAAACCGCGAAGCGCAAG -3' |
| | | |
| AAV-4: | (cap, links) | 5'- CCAGATATGACTGACGGTTACCTTCC -3' |
| | (cap, rechts) | 5'- GTCCAATCGATGCAGTTGTAAACCGCGAAGCGCAAG -3' |
| | (rep, links) | 5'- CACTGACGTCAATGTGACGTCCTAGG -3' |
| | (rep, rechts) | 5'- CGTGACCTCCTTGACCTGGATGTTG -3' |
| | | |
| AAV-5 : | (links) | 5'- GGAAAACTTGTCAGATTTTGG -3' |
| | (rechts) | 5'- GTCCAATCGATGCCACAAGAGGCAGTATTTTACTGAC -3' |
| | | |
| AAV-6: | (links) | 5'- CTGGATGACTGTGTTTCTGAGC -3' |
| | (rechts) | 5'- GTCCAATCGATGCGAAGCGCAACTAAGCAG -3' |

## Patentansprüche

1. AAV-Helferplasmid, das folgende DNA-Sequenzen umfaßt:
(a1) Das *rep*-Gen von AAV-2; und
(a2) das *cap*-Gen von AAV-1, AAV-3, AAV-4, AAV-5 oder AAV-6; oder
(b) das *cap*-Gen und das *rep-*Gen von jeweils AAV-1, AAV-3, AAV-4, AAV-5 oder AAV-6; und
(c) alle weiteren Helfervirus-DNA-Sequenzen, die für eine Ausbildung von AAV-Partikeln notwendig sind, wobei diese Helfervirus-DNA-Sequenzen die Ad5-Gene E2A, E4 und VA sind.

2. AAV-Helferplasmid nach Anspruch 1, das zusätzlich eine Expressionskasette zur Expression eines Fluoreszenzproteins enthält.

3. AAV-Helferplasmid nach Anspruch 2, wobei das Fluoreszenzprotein das "red fluorescent" Protein ist.

4. AAV-Helferplasmid nach Anspruch 2 oder 3, wobei das Fluoreszenzprotein mit einem RSV-Promotor funktionell verknüpft ist.

5. AAV-Helferplasmid nach Anspruch 4, das pDP1 mit der Hinterlegungsnummer DSM 14256, pDP3 mit der Hinterlegungsnummer DSM 14255, pDP4 mit der Hinterlegungsnummer DSM 14254, pDP5 mit der Hinterlegungsnummer DSM 14253 oder pDP6 mit der Hinterlegungsnummer DSM 14252 ist.

6. AAV-Partikel, dessen Kapsidhülle von dem AAV-Helferplasmid nach einem der Ansprüche 1 bis 5 kodiert wird und das einen AAV-Expressionsvektor enthält.

7. AAV-Partikel nach Anspruch 6, wobei der AAV-Expressionsvektor mindestens folgende DNA-Sequenzen umfaßt:
(a) die 5'ITR und 3'ITR eines AAV-2;
(b) einen in Säugern aktiven konstitutiven oder induzierbaren Promotor und
(c) ein Poly-Adenylierungssignal.

8. Arzneimittel, ein AAV-Helferplasmid nach einem der Ansprüche 1 bis 5 oder ein AAV-Partikel nach Anspruch 6 oder 7 und einen pharmazeutisch verträglichen Träger enthaltend.

9. AAV-Helferplasmid nach einem der Ansprüche 1 bis 5 oder ein AAV-Partikel nach Anspruch 6 oder 7 für die Gentherapie.

10. Säugerzelle, das AAV-Partikel nach Anspruch 6 oder 7 enthaltend.

11. Säugerzelle nach Anspruch 10, die eine 293-Zelle ist.

12. Verfahren zur Herstellung eines AAV-Partikels, **dadurch gekennzeichnet, daß** man Säugerzellen mit einem AAV-Helferplasmid nach einem der Ansprüche 1 bis 5 oder einem in Anspruch 6 oder 7 definierten AAV-Expressionsvektor transfiziert und nach Züchtung der Zellen das AAV-Partikel aus den Säugerzellen oder dem Medium isoliert.

## Claims

1. An AAV helper plasmid comprising the following DNA sequences:
(a1) the *rep* gene of AAV-2; and
(a2) the *cap* gene of AAV-1, AAV-3, AAV-4, AAV-5 or AAV6; or
(b) the *cap* gene and the *rep* gene of AAV-1, AAV-3, AAV-4, AAV-5 or AAV-6 each; and
(c) all further helper virus DNA sequences necessary for forming AAV particles, said helper virus DNA sequences being the Ad5 genes E2A, E4 and VA.

2. The AAV helper plasmid according to claim 1, which additionally contains an expression cassette for the expression of a fluorescent protein.

3. The AAV helper plasmid according to claim 2, wherein the fluorescent protein is the "red fluorescent" protein.

4. The AAV helper plasmid according to claim 2 or 3, wherein the fluorescent protein is functionally linked to an RSV promoter.

5. The AAV helper plasmid according to claim 4, which is pDP1 with accession number DSM 14256, pDP3 with accession number DSM 14255, pDP4 with accession number DSM 14254, pDP5 with accession number DSM 14253 or pDP6 with accession number DSM 14252.

6. An AAV particle whose capsid coat is encoded by the AAV helper plasmid according to any of claims 1 to 5 and which contains an AAV expression vector.

7. The AAV particle according to claim 6, wherein the AAV expression vector comprises at least the following DNA sequences:
(a) the 5'ITR and 3'ITR of an AAV-2;
(b) a constitutive or inducible promoter active in mammals, and
(c) a polyadenylation signal.

8. A medicament containing an AAV helper plasmid according to any of claims 1 to 5 or an AAV particle according to claim 6 or 7 and a pharmaceutically compatible carrier.

9. An AAV helper plasmid according to any of claims 1 to 5 or an AAV particle according to claim 6 or 7 for gene therapy.

10. A mammalian cell containing the AAV particle according to claim 6 or 7.

11. The mammalian cell according to claim 10, which is a 293 cell.

12. A method of producing an AAV particle, **characterized in that** mammalian cells are transfected with an AAV helper plasmid according to any of claims 1 to 5 or an AAV expression vector defined in claim 6 or 7 and after culturing the cells the AAV particle is isolated from the mammalian cells or the medium.

## Revendications

1. Plasmide auxiliaire AAV, comprenant les séquences d'ADN suivantes:
(a1) le gène rep d'AAV-2; et
(a2) le gène cap d'AAV-1, d'AAV-3. d'AAV-4, d'AAV-5 ou d'AAV-6; ou
(b) le gène cap et le gène rep d'AAV-1, d'AAV-3, d'AAV-4, d'AAV-5 ou d'AAV-6, respectivement; et
(c) toutes les autres séquences d'ADN de virus auxiliaire qui sont nécessaires pour une formation de particules d'AAV, ces séquences d'ADN de virus auxiliaire étant les gènes Ad5 E2A, E4 et VA.

2. Plasmide auxiliaire AAV selon la revendication 1, contenant en outre une cassette d'expression pour l'expression d'une protéine de fluorescence.

3. Plasmide auxiliaire AAV selon la revendication 2, la protéine de fluorescence étant la protéine de "fluorescence rouge".

4. Plasmide auxiliaire AAV selon la revendication 2 ou 3, la protéine de fluorescence étant couplée fonctionnellement à un promoteur RSV.

5. Plasmide auxiliaire AAV selon la revendication 4, qui est pDP1 au numéro de dépôt DSM 14256, pDP3 au numéro de dépôt DSM 14255, pDP4 au numéro de dépôt DSM 14254, pDP5 au numéro de dépôt DSM 14253 ou pDP6 au numéro de dépôt DSM 14252.

6. Particule d'AAV, dont l'enveloppe de capside est codée par le plasmide auxiliaire AAV selon l'une des revendications 1 à 5 et qui contient un vecteur d'expression d'AAV.

7. Particule d'AAV selon la revendication 6, le vecteur d'expression d'AAV comprenant au moins les séquences d'ADN suivantes:
(a) les 5'ITR et 3'ITR d'un AAV-2;
(b) un promoteur constitutif ou inductible actif dans les mammifères; et
(c) un signal de polyadénylation.

8. Médicament, contenant un plasmide auxiliaire AAV selon l'une des revendications 1 à 5 ou une particule d'AAV selon la revendication 6 ou 7 et un porteur pharmaceutiquement tolérable.

9. Plasmide auxiliaire AAV selon l'une des revendications 1 à 5 ou une particule d'AAV selon la revendication 6 ou 7 pour la thérapie génique.

10. Cellule de mammifère, contenant la particule d'AAV selon la revendication 6 ou 7.

11. Cellule de mammifère selon la revendication 10, qui est une cellule 293.

12. Procédé pour produire une particule d'AAV, **caractérisé par le fait que** l'on transfecte des cellules de mammifère avec un plasmide auxiliaire AAV selon l'une des revendications 1 à 5 ou un vecteur d'expression d'AAV défini dans la revendication 6 ou 7 et que l'on isole, après la culture des cellules, la particule d'AAV des cellules de mammifère ou du bouillon de culture.
